Europäisches Patentamt

European Patent Office

Office européen des brevets

(19)

(11) Publication number: **0 265 874**
**A2**

# (12) EUROPEAN PATENT APPLICATION

(21) Application number: 87115600.6

(22) Date of filing: 23.10.87

(51) Int. Cl.⁴: **C12N 15/00 , C12N 9/72**

The applicant has filed a statement in accordance with Rule 28 (4) EPC (issue of a sample only to an expert). Accession number(s) of the deposit(s): ATCC 37146.

(30) Priority: 23.10.86 JP 253078/86

(43) Date of publication of application:
04.05.88 Bulletin 88/18

(84) Designated Contracting States:
BE CH DE ES FR GB LI NL SE

(71) Applicant: **THE GREEN CROSS CORPORATION**
**15-1, Imabashi-1-chome Higashi-ku**
**Osaka-shi**
**Osaka 541(JP)**

(72) Inventor: **Amatsuji, Yasuo**
**6-2, Nagao Motomachi 1-chome**
**Hirakata-shi Osaka(JP)**
Inventor: **Okabayashi, Ken**
**1-6-24, Kuzuha Asahi 1-chome**
**Hirakata-shi Osaka(JP)**
Inventor: **Nagai, Masanori**
**10-10, Nishiyama Waki**
**Yahata-shi Kyoto(JP)**
Inventor: **Arimura, Hirofumi**
**18-1-401, Uenosaka 2-chome**
**Toyonaka-shi Osaka(JP)**
Inventor: **Suyama, Tadakazu**
**3-7, Matsuigaoka 4-chome Tanabe-cho**
**Tsuzuki-gun Kyoto(JP)**

(74) Representative: **Hansen, Bernd, Dr.rer.nat. et al**
**Hoffmann, Eitle & Partner Patentanwälte**
**Arabellastrasse 4**
**D-8000 München 81(DE)**

(54) Method of producing human prourokinase.

(57) A method of producing glycosylated single-chain prourokinase which comprises cultivating dihydrofolate reductase gene-deficient CHO-K1 cells transformed with a plasmid containing, as inserts a pro-UK cDNA sequence, prepared by using human kidney cell line-derived mRNA as template, SV40 promoter genes, and the dihydrofolate reductase gene, wherein the expression of said pro-UK cDNA is controlled by a SV40 promoter gene and the expression of said dihydrofolate reductase gene is controlled by a second SV40 promoter gene.

EP 0 265 874 A2

## METHOD OF PRODUCING HUMAN PROUROKINASE

### FIELD OF THE INVENTION

This invention relates to a method of producing single-chain prourokinase (hereinafter referred to as "pro-UK") in a glycosylated form. More particularly, the invention relates to a method of producing glycosylated pro-UK which comprises cultivating dihydrofolate reductase (hereinafter abbreviated as "dhfr") gene-deficient CHO-K1 cells transformed with a plasmid which contains, as inserts, a pro-UK cDNA obtained on the basis of mRNA obtained from a human kidney cell line, SV40 promoter genes, and the dhfr gene.

### BACKGROUND OF THE INVENTION

Urokinase (hereinafter abbreviated as "UK") is a plasminogen activator obtained from human urine. It is widely used in the treatment of various thrombotic or obstructive diseases and also in combination with anticancer agents, among others, and produces good therapeutic effects. At present, urokinase is produced mainly by purification from human urine (cf. Williams, J.R.B., Brit. J. Exp. Pathol., 32:530, 1951).

As a result of technological development in the area of cell culture in search of a substitute method, a method comprising isolating human kidney cells, subcultur ing them and recovering urokinase has become an important method for obtaining UK. Meanwhile, recent improvements in recombinant DNA technology have made it possible to produce desired proteins on a commercial scale using such microorganisms as Escherichia coli, yeasts and Bacillus subtilis [cf. Goeddel, D.V., Itakura, K., et al., Proc. Natol. Acad. Sci. USA, 76, 106 (1979) and Nagata, S., Taira, S.H. and Wissmarn, C., Nature, 284 :1316 (1980)].

### SUMMARY OF THE INVENTION

The present inventors made investigations in an attempt to produce urokinase using recombinant DNA technology. As a result, mRNA from a human kidney cells was obtained from which a cDNA sequence having the following sequence (I) using said mRNA as template was obtained, and further glycosylated pro-UK by cultivating dhfr gene-deficient CHO-K1 cells transformed with a plasmid in which said cDNA, SV40 promoter genes and the dhfr gene are inserted, wherein the expression of said cDNA is controlled by a SV40 promoter gene and the expression of said dhfr gene is controlled by a second SV40 promoter gene.

Sequence (I): Base sequence of the structural gene for pro-UK

```
 1                                      10                                     20
Ser-Asn-Glu-Leu-His-Gln-Val-Pro-Ser-Asn-Cys-Asp-Cys-Leu-Asn-Gly-Gly-Thr-Cys-Val-
AGC AAT GAA CTT CAT CAA GTT CCA TCG AAC TGT GAC TGT CTA AAT GGA GGA ACA TGT GTG

21                                      30                                     40
Ser-Asn-Lys-Tyr-Phe-Ser-Asn-Ile-His-Trp-Cys-Asn-Cys-Pro-Lys-Lys-Phe-Gly-Gly-Gln-
TCC AAC AAG TAC TTC TCC AAC ATT CAC TGG TGC AAC TGC CCA AAG AAA TTC GGA GGG CAG

41                                      50                                     60
His-Cys-Glu-Ile-Asp-Lys-Ser-Lys-Thr-Cys-Tyr-Glu-Gly-Asn-Gly-His-Phe-Tyr-Arg-Gly-
CAC TGT GAA ATA GAT AAG TCA AAA ACC TGC TAT GAG GGG AAT GGT CAC TTT TAC CGA GGA

61                                      70                                     80
Lys-Ala-Ser-Thr-Asp-Thr-Met-Gly-Arg-Pro-Cys-Leu-Pro-Trp-Asn-Ser-Ala-Thr-Val-Leu-
AAG GCC AGC ACT GAC ACC ATG GGC CGG CCC TGC CTG CCC TGG AAC TCT GCC ACT GTC CTT

81                                      90                                     100
Gln-Gln-Thr-Tyr-His-Ala-His-Arg-Ser-Asp-Ala-Leu-Gln-Leu-Gly-Leu-Gly-Lys-His-Asn-
CAG CAA ACG TAC CAT GCC CAC AGA TCT GAT GCT CTT CAG CTG GGC CTG GGG AAA CAT AAT

101                                     110                                    120
Tyr-Cys-Arg-Asn-Pro-Asp-Asn-Arg-Arg-Arg-Pro-Trp-Cys-Tyr-Val-Gln-Val-Gly-Leu-Lys-
TAC TGC AGG AAC CCA GAC AAC CGG AGG CGA CCC TGG TGC TAT GTG CAG GTG GGC CTA AAG

121                                     130                                    140
Pro-Leu-Val-Gln-Glu-Cys-Met-Val-His-Asp-Cys-Ala-Asp-Gly-Lys-Lys-Pro-Ser-Ser-Pro-
CCG CTT GTC CAA GAG TGC ATG GTG CAT GAC TGC GCA GAT GGA AAA AAG CCC TCC TCT CCT

141                                     150                                    160
Pro-Glu-Glu-Leu-Lys-Phe-Gln-Cys-Gly-Gln-Lys-Thr-Leu-Arg-Pro-Arg-Phe-Lys-Ile-Ile-
CCA GAA GAA TTA AAA TTT CAG TGT GGC CAA AAG ACT CTG AGG CCC CGC TTT AAG ATT ATT

161                                     170                                    180
Gly-Gly-Glu-Phe-Thr-Thr-Ile-Glu-Asn-Gln-Pro-Trp-Phe-Ala-Ala-Ile-Tyr-Arg-Arg-His-
GGG GGA GAA TTC ACC ACC ATC GAG AAC CAG CCC TGG TTT GCG GCC ATC TAC AGG AGG CAC

181                                     190                                    200
Arg-Gly-Gly-Ser-Val-Thr-Tyr-Val-Cys-Gly-Gly-Ser-Leu-Ile-Ser-Pro-Cys-Trp-Val-Ile-
CGG GGG GGC TCT GTC ACC TAC GTG TGT GGA GGC AGC CTC ATC AGC CCT TGC TGG GTG ATC

201                                     210                                    220
Ser-Ala-Thr-His-Cys-Phe-Ile-Asp-Tyr-Pro-Lys-Lys-Glu-Asp-Tyr-Ile-Val-Tyr-Leu-Gly-
AGC GCC ACA CAC TGC TTC ATT GAT TAC CCA AAG AAG GAG GAC TAC ATC GTC TAC CTG GGT

221                                     230                                    240
Arg-Ser-Arg-Leu-Asn-Ser-Asn-Thr-Gln-Gly-Glu-Met-Lys-Phe-Glu-Val-Glu-Asn-Leu-Ile-
CGC TCA AGG CTT AAC TCC AAC ACG CAA GGG GAG ATG AAG TTT GAG GTG GAA AAC CTC ATC

241                                     250                                    260
Leu-His-Lys-Asp-Tyr-Ser-Ala-Asp-Thr-Leu-Ala-His-His-Asn-Asp-Ile-Ala-Leu-Leu-Lys-
CTA CAC AAG GAC TAC AGC GCT GAC ACG CTT GCT CAC CAC AAC GAC ATT GCC TTG CTG AAG

261                                     270                                    280
Ile-Arg-Ser-Lys-Glu-Gly-Arg-Cys-Ala-Gln-Pro-Ser-Arg-Thr-Ile-Gln-Thr-Ile-Cys-Leu-
ATC CGT TCC AAG GAG GGC AGG TGT GCG CAG CCA TCC CGG ACT ATA CAG ACC ATC TGC CTG

281                                     290                                    300
Pro-Ser-Met-Tyr-Asn-Asp-Pro-Gln-Phe-Gly-Thr-Ser-Cys-Glu-Ile-Thr-Gly-Phe-Gly-Lys-
CCC TCG ATG TAT AAC GAT CCC CAG TTT GGC ACA AGC TGT GAG ATC ACT GGC TTT GGA AAA
```

3

```
301                                      310                                      320
Glu-Asn-Ser-Thr-Asp-Tyr-Leu-Tyr-Pro-Glu-Gln-Leu-Lys-Met-Thr-Val-Val-Lys-Leu-Ile-
GAG AAT TCT ACC GAC TAT CTC TAT CCG GAG CAG CTG AAG ATG ACT GTT GTG AAG CTG ATT

321                                      330                                      340
Ser-His-Arg-Glu-Cys-Gln-Gln-Pro-His-Tyr-Tyr-Gly-Ser-Glu-Val-Thr-Thr-Lys-Met-Leu-
TCC CAC CGG GAG TGT CAG CAG CCC CAC TAC TAC GGC TCT GAA GTC ACC ACC AAA ATG CTG

341                                      350                                      360
Cys-Ala-Ala-Asp-Pro-Gln-Trp-Lys-Thr-Asp-Ser-Cys-Gln-Gly-Asp-Ser-Gly-Gly-Pro-Leu-
TGT GCT GCT GAC CCA CAG TGG AAA ACA GAT TCC TGC CAG GGA GAC TCA GGG GGA CCC CTC

361                                      370                                      380
Val-Cys-Ser-Leu-Gln-Gly-Arg-Met-Thr-Leu-Thr-Gly-Ile-Val-Ser-Trp-Gly-Arg-Gly-Cys-
GTC TGT TCC CTC CAA GGC CGC ATG ACT TTG ACT GGA ATT GTG AGC TGG GGC CGT GGA TGT

381                                      390                                      400
Ala-Leu-Lys-Asp-Lys-Pro-Gly-Val-Tyr-Thr-Arg-Val-Ser-His-Phe-Leu-Pro-Trp-Ile-Arg-
GCC CTG AAG GAC AAG CCA GGC GTC TAC ACG AGA GTC TCA CAC TTC TTA CCC TGG ATC CGC

401                                      410
Ser-His-Thr-Lys-Glu-GLu-Asn-Gly-Leu-Ala-Leu
AGT CAC ACC AAG GAA GAG AAT GGC CTG GCC CTC
```

The invention thus provides a method of producing glycosylated single-chain prourokinase which comprises cultivating dihydrofolate reductase gene-deficient CHO-K1 cells transformed with a plasmid containing, as inserts, the above cDNA prepared using human kidney cell line-derived mRNA as template, the SV40 promoter gene and the dihydrofolate reductase gene.

## BRIEF DESCRIPTION OF THE DRAWINGS

Fig. 1 shows a construction scheme for pSVD-UK, an expression plasmid. In the figure, pre-UK indicates the DNA coding for pro-UK, dhfr the dihydrofolate reductase gene, SV40 ori the SV40-derived enhancer/promotor region, ▨▨▨ the SV40 polyadenylation signal, and → the direction of transcription.

Fig. 2 shows a ligation flowchart for pcDV₁ and pL₁ to obtain pSV-G₁.

Fig. 3 shows the restriction enzyme cleavage map of pSV-G₁.

Fig. 4 shows the restriction enzyme cleavage map of pSV-G₁-pre-UK. In the figure, E indicates the enhancer, P the promoter, SJ the splicing junction, and A(n) the SV40 polyadenylation signal.

Fig. 5 shows the structure of the neighborhood of the pre-UK cDNA in pSV-G₁-pre-UK. In the figure, NC indicates the 5′ noncoding region, SS the signal sequence, and SJ the splicing junction.

Fig. 6 shows the pSV2-dhfr plasmid. (ATCC 37146).

## DETAILED DESCRIPTION OF THE INVENTION

As a plasmid containing a pro-UK gene represented by Sequence (I) obtained on the basis of mRNA isolated from a human kidney cell line, pUK33 as described in EP 154272A can be used.

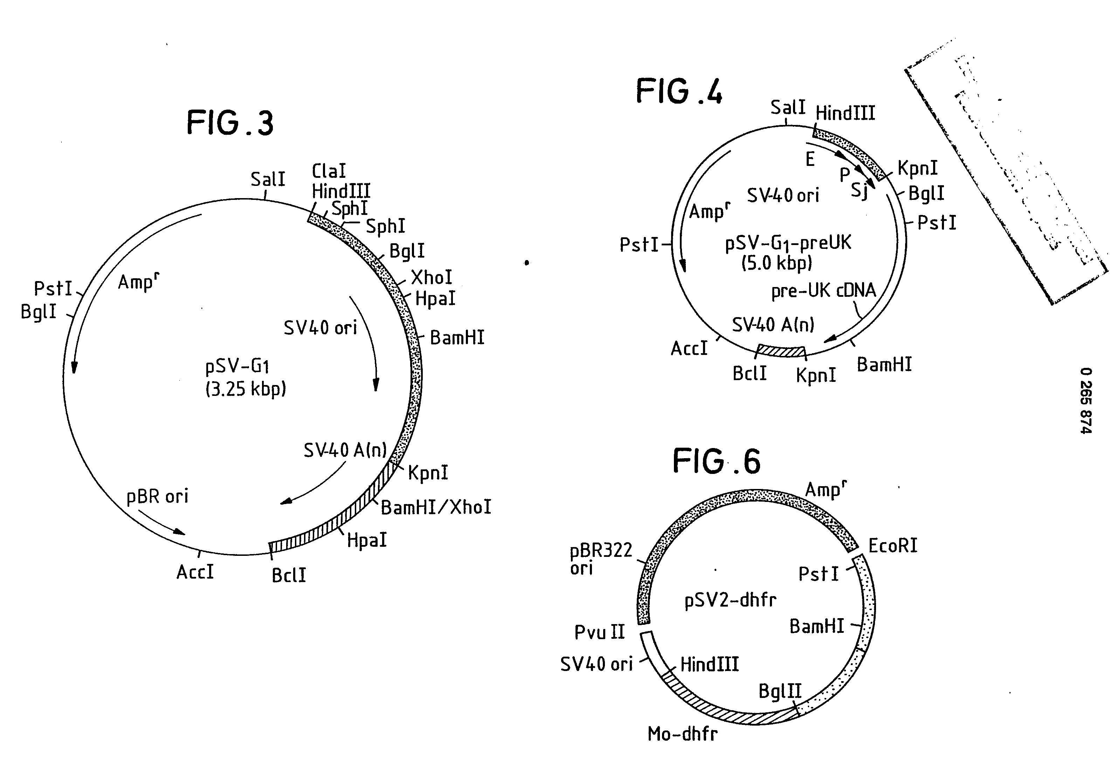

A suitable example of SV40 promoter genes used in the present invention is one as described in EP 154272A and should not be limited thereto. pSV-G₁ (Fig. 3) can be preferably used in the present invention as plasmid containing the SV40 promoter gene inserted therein.

As a dhfr gene, those known in the art, as described in EP 117060A can be used in the present invention and it is should not be limited thereto. A suitable example of a plasmid containing the dhrf gene inserted therein is pSV2-dhfr (ATCC 37146, Fig. 6).

Examples of a plasmid vector in which a pro-UK cDNA, SV40 promoter genes and a dhfr gene are to be inserted are preferably those derived from pBR322 or SV40.

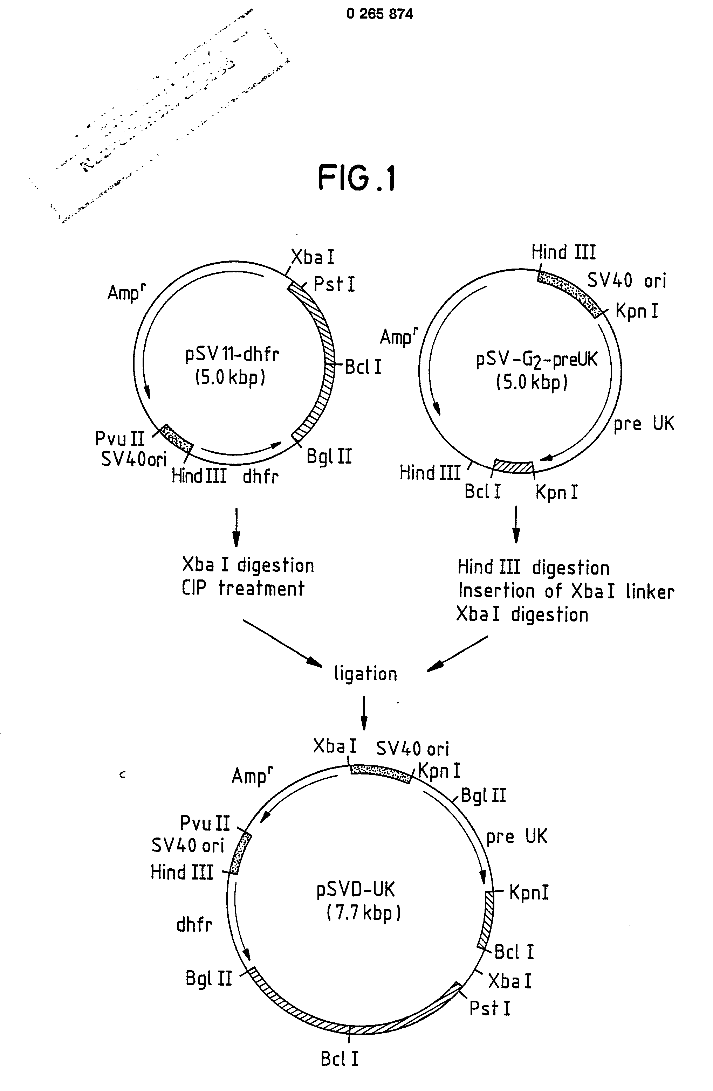

The above-described recombinant plasmid according to the present invention is produced by inserting the pro-UK cDNA fragment into the SV40 promoter-containing plasmid at the downstream from the SV40 promoter region and ligating the thus-obtained recombinant DNA with the dhfr gene-containing plasmid so that the pro-UK gene and the dhfr gene are inserted in opposite directions (Fig. 1).

As a host cell, CHO-K1 cell-derived dhfr gene-deficient cells can be used in the present invention. A suitable example thereof is DXB-11 cell provided by Dr. L. Chasin at Columbia University, USA ** The cells are subcultured using HamF 12 medium (Nissui Pharmaceutical) containing 10% fetal calf serum. For dissociation and dispersion of cells, a 0.1% collagenase solution in phosphate buffer is used.

Transformation of the host cells can be carried out by the conventional method such as a calcium phosphate precipitate mehtod, a protoplast fusion method, an electropolation method and the like.

The transformants can be cultured in the medium conventionally used for the host cells per se. Examples of the medium include DMEM (Dulbecco modified Eagle's medium), RPMI-1640 medium and the like.

Cultivation of the transformants are carried out at a temperature of 15 to 43°C, preferably about 37°C, for about 10 to 60 days aeration or with stirring, if desired.

The desired product, glycosylated single-chain prourokinase, according to the present invention can be recovered from culture supernatants and purified by the conventional method such as affinity chromatography, fractionation, and the like.

A method of producing glycosylated single-chain pro-UK according to the present invention is illustrated in detail below.

(1) <u>Preparation of a DNA sequence (pre-UK cDNA) coding for pro-UK</u>

A pre-UK cDNA-containing plasmid, pUK33, is obtained by the method described in EP 154272A, incorporated by reference herein. The cDNA was obtained using urokinase mRNA isolated and purified from a human kidney cell line.

As a result, it has been established that urokinase is composed of 20 amino acids, which constitute a signal peptide, and succeeding 411 amino acids. In view of the DNA base sequence of the urokinase cDNA, among others, it has also been revealed that there is a 5′ noncoding region of 80 bases or more and a 3′ noncoding region of 800 bases or more in the urokinase mRNA [see Sequence (II) below].

**(as described in Proc. Natl. Acad. Sci., USA, 77, 7 4216-4220 (1980)).

Sequence (II): Base sequence of the pro-UK structural gene, signal sequence and noncoding region, and the corresponding amino acid sequence.

.5' TCCACCTGTCCCCGCAGCGCCGGCTCGCGCCCTCCTGCCGCAGCCACCGAGCCGCCGTCTACCGCCCCGACCTCGCCACC

```
          -20                                   -10                                      -1
          Met Arg Ala Leu Leu Ala Arg Leu Leu Leu Cys Val Leu Val Val Ser Asp Ser Lys Gly
          ATG TGA GCC CTG CTG GCG CGC CTG CTT CTC TGC GTC CTG GTC GTG AGC GAC TCC AAA GGC

          1                                     10                                       20
          Ser-Asn-Glu-Leu-His-Gln-Val-Pro-Ser-Asn-Cys-Asp-Cys-Leu-Asn-Gly-Gly-Thr-Cys-Val-
          AGC AAT GAA CTT CAT CAA GTT CCA TCG AAC TGT GAC TGT CTA AAT GGA GGA ACA TGT GTG

          21                                    30                                       40
          Ser-Asn-Lys-Tyr-Phe-Ser-Asn-Ile-His-Trp-Cys-Asn-Cys-Pro-Lys-Lys-Phe-Gly-Gly-Gln-
          TCC AAC AAG TAC TTC TCC AAC ATT CAC TGG TGC AAC TGC CCA AAG AAA TTC GGA GGG CAG

          41                                    50                                       60
          His-Cys-Glu-Ile-Asp-Lys-Ser-Lys-Thr-Cys-Tyr-Glu-Gly-Asn-Gly-His-Phe-Tyr-Arg-Gly-
          CAC TGT GAA ATA GAT AAG TCA AAA ACC TGC TAT GAG GGG AAT GGT CAC TTT TAC CGA GGA

          61                                    70                                       80
          Lys-Ala-Ser-Thr-Asp-Thr-Met-Gly-Arg-Pro-Cys-Leu-Pro-Trp-Asn-Ser-Ala-Thr-Val-Leu-
          AAG GCC AGC ACT GAC ACC ATG GGC CGG CCC TGC CTG CCC TGG AAC TCT GCC ACT GTC CTT

          81                                    90                                       100
          Gln-Gln-Thr-Tyr-His-Ala-His-Arg-Ser-Asp-Ala-Leu-Gln-Leu-Gly-Leu-Gly-Lys-His-Asn-
          CAG CAA ACG TAC CAT GCC CAC AGA TCT GAT GCT CTT CAG CTG GGC CTG GGG AAA CAT AAT

          101                                   110                                      120
          Tyr-Cys-Arg-Asn-Pro-Asp-Asn-Arg-Arg-Arg-Pro-Trp-Cys-Tyr-Val-Gln-Val-Gly-Leu-Lys-
          TAC TGC AGG AAC CCA GAC AAC CGG AGG CGA CCC TGG TGC TAT GTG CAG GTG GGC CTA AAG

          121                                   130                                      140
          Pro-Leu-Val-Gln-Glu-Cys-Met-Val-His-Asp-Cys-Ala-Asp-Gly-Lys-Lys-Pro-Ser-Ser-Pro-
          CCG CTT GTC CAA GAG TGC ATG GTG CAT GAC TGC GCA GAT GGA AAA AAG CCC TCC TCT CCT

          141                                   150                                      160
          Pro-Glu-Glu-Leu-Lys-Phe-Gln-Cys-Gly-Gln-Lys-Thr-Leu-Arg-Pro-Arg-Phe-Lys-Ile-Ile-
          CCA GAA GAA TTA AAA TTT CAG TGT GGC CAA AAG ACT CTG AGG CCC CGC TTT AAG ATT ATT
```

```
      161                              170                                    180
      Gly-Gly-Glu-Phe-Thr-Thr-Ile-Glu-Asn-Gln-Pro-Trp-Phe-Ala-Ala-Ile-Tyr-Arg-Arg-His-
      GGG GGA GAA TTC ACC ACC ATC GAG AAC CAG CCC TGG TTT GCG GCC ATC TAC AGG AGG CAC

      181                              190                                    200
      Arg-Gly-Gly-Ser-Val-Thr-Tyr-Val-Cys-Gly-Gly-Ser-Leu-Ile-Ser-Pro-Cys-Trp-Val-Ile-
      CGG GGG GGC TCT GTC ACC TAC GTG TGT GGA GGC AGC CTC ATC AGC CCT TGC TGG GTG ATC

      201                              210                                    220
      Ser-Ala-Thr-His-Cys-Phe-Ile-Asp-Tyr-Pro-Lys-Lys-Glu-Asp-Tyr-Ile-Val-Tyr-Leu-Gly-
      AGC GCC ACA CAC TGC TTC ATT GAT TAC CCA AAG AAG GAG GAC TAC ATC GTC TAC CTG GGT

      221                              230                                    240
      Arg-Ser-Arg-Leu-Asn-Ser-Asn-Thr-Gln-Gly-Glu-Met-Lys-Phe-Glu-Val-Glu-Asn-Leu-Ile-
      CGC TCA AGG CTT AAC TCC AAC ACG CAA GGG GAG ATG AAG TTT GAG GTG GAA AAC CTC ATC

      241·                             250                                    260
      Leu-His-Lys-Asp-Tyr-Ser-Ala-Asp-Thr-Leu-Ala-His-His-Asn-Asp-Ile-Ala-Leu-Leu-Lys-
      CTA CAC AAG GAC TAC AGC GCT GAC ACG CTT GCT CAC CAC AAC GAC ATT GCC TTG CTG AAG

      261                              270                                    280
      Ile-Arg-Ser-Lys-Glu-Gly-Arg-Cys-Ala-Gln-Pro-Ser-Arg-Thr-Ile-Gln-Thr-Ile-Cys-Leu-
      ATC CGT TCC AAG GAG GGC AGG TGT GCG CAG CCA TCC CGG ACT ATA CAG ACC ATC TGC CTG

      281                              290                                    300
      Pro-Ser-Met-Tyr-Asn-Asp-Pro-Gln-Phe-Gly-Thr-Ser-Cys-Glu-Ile-Thr-Gly-Phe-Gly-Lys-
      CCC TCG ATG TAT AAC GAT CCC CAG TTT GGC ACA AGC TGT GAG ATC ACT GGC TTT GGA AAA

      301                              310                                    320
      Glu-Asn-Ser-Thr-Asp-Tyr-Leu-Tyr-Pro-Glu-Gln-Leu-Lys-Met-Thr-Val-Val-Lys-Leu-Ile-
      GAG AAT TCT ACC GAC TAT CTC TAT CCG GAG CAG CTG AAG ATG ACT GTT GTG AAG CTG ATT

      321                              330                                    340
      Ser-His-Arg-Glu-Cys-Gln-Gln-Pro-His-Tyr-Tyr-Gly-Ser-Glu-Val-Thr-Thr-Lys-Met-Leu-
      TCC CAC CGG GAG TGT CAG CAG CCC CAC TAC TAC GGC TCT GAA GTC ACC ACC AAA ATG CTG

      341                              350                                    360
      Cys-Ala-Ala-Asp-Pro-Gln-Trp-Lys-Thr-Asp-Ser-Cys-Gln-Gly-Asp-Ser-Gly-Gly-Pro-Leu-
      TGT GCT GCT GAC CCA CAG TGG AAA ACA GAT TCC TGC CAG GGA GAC TCA GGG GGA CCC CTC

      361                              370                                    380
      Val-Cys-Ser-Leu-Gln-Gly-Arg-Met-Thr-Leu-Thr-Gly-Ile-Val-Ser-Trp-Gly-Arg-Gly-Cys-
      GTC TGT TCC CTC CAA GGC CGC ATG ACT TTG ACT GGA ATT GTG AGC TGG GGC CGT GGA TGT

      381                              390                                    400
      Ala-Leu-Lys-Asp-Lys-Pro-Gly-Val-Tyr-Thr-Arg-Val-Ser-His-Phe-Leu-Pro-Trp-Ile-Arg
      GCC CTG AAG GAC AAG CCA GGC GTC TAC ACG AGA GTC TCA CAC TTC TTA CCC TGG ATC CGC

      401                              410
      Ser-His-Thr-Lys-Glu-GLu-Asn-Gly-Leu-Ala-Leu Stop
      AGT CAC ACC AAG GAA GAG AAT GGC CTG GCC CTC TGA GGGTCCCCAGGGAGGAAACGGGCACCACCCGC

      TTTCTTGCTGGTTGTCATTTTTGCAGTAGAGTCATCTCCATCAGCTGTAAGAAGAGACTGGGAAGA  3'
```

(2) Introduction of the SV40 gene promoter into a plasmid

The SV40 gene promoter was utilized as an appropriate promoter in the practice of the invention.

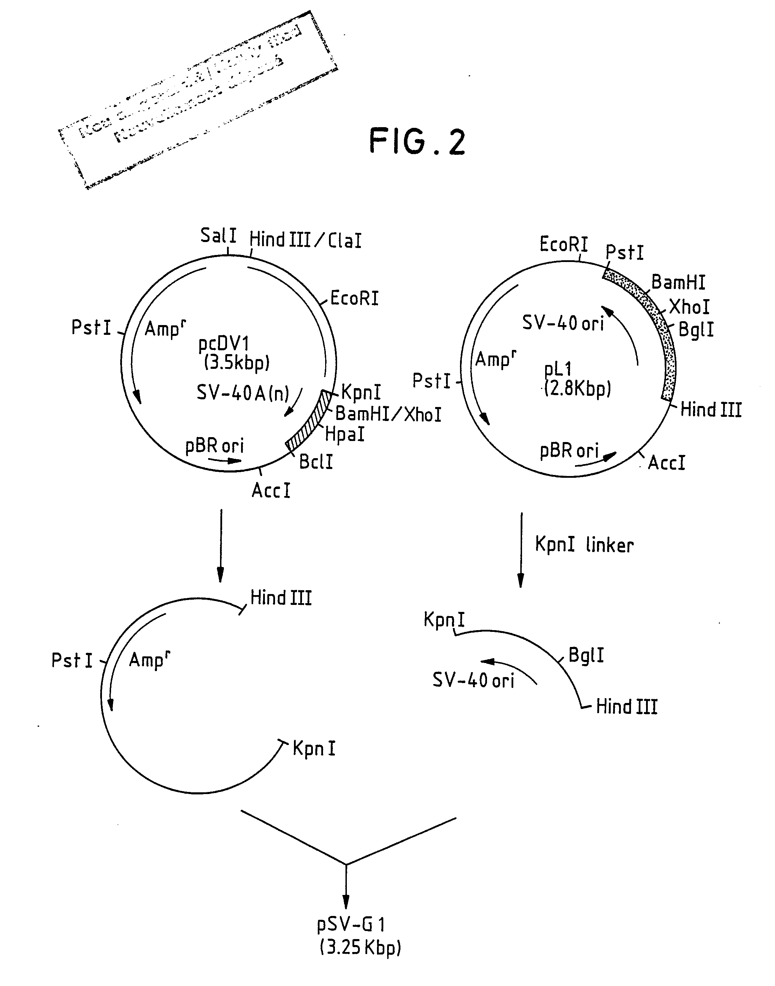

A plasmid PSV-G₁ (Fig. 3), was constructed using, as starting plasmids pcDV₁ and pL₁ (Fig. 2) produced as described by Okayama and Berg, Molec. Cell. Biolo., 3:280-289 (1983). pcDV₁ and pL₁ are hybrid plasmids between pBR322 and an SV40 DNA. They were obtained from PL Biochemicals (Pharmacia). To facilitate the insertion of the urokinase-encoding DNA into these plasmids, pSV-G₁ was constructed by inserting the HindIII-PstI fragment of pL₁ into pcDV₁ at the HindIII-KpnI site thereof.

More specifically, 4 µg of pL₁ was digested with PstI and then the protruding 3′ ends were converted to blunt ends with T₄ DNA polymerase using 76 µl of reaction mixture No. 1 (4 µg of pL₁, 8 µl of 10 × T₄ polymerase buffer, 4.0 µl of 2 mM dNTP solution in distilled water) which was heated to 65°C for 3 minutes and then quenched. Then, 4 µl of T₄ polymerase (10 U) was added to make the total volume 80 µl, and the reaction was performed at 37°C for 5 minutes. Thereafter, 8 µl of 0.25 M EDTA (pH 8.0) and 80 µl of distilled water were added and, after non phenol extraction, the aqueous layer was recovered. The DNA was

7

recovered by precipitation with ethanol, dried under reduced pressure, and subjected to KpnI linker ligation. For the ligation reaction, 18 $\mu$l of reaction mixture No. 2 [4 $\mu$g of dried DNA, 2 $\mu$l (2 $\mu$g) of 5'-phosphorylated KpnI linker, 0.2 $\mu$l of 10 $\times$ ligase buffer in distilled water) was heated at 65°C for 3 minutes and then gradually cooled. Next, 2 $\mu$l (5 U) of T4 ligase was added, and the reaction was performed overnight at 16°C. Thereafter, digestion was carried out with KpnI and then with HindIII. The digestion products were subjected to 5% polyacrylamide gel electro-phoresis (PAGE) and a DNA fragment of about 600 bp was recovered.

Separately, pcDV₁ was digested with HindIII and KpnI, and a DNA fragment of about 2.7 kbp was isolated on 1% agarose gel. A 100 ng portion of the fragment was ligated with the above-mentioned, pL₁-derived DNA fragment of about 600 bp. For effecting the ligation reaction, 2 $\mu$l (5.6 U) of T4 ligase were added to 8 $\mu$l of reaction mixture No. 3 (150 ng of pL₁ fragment DNA, 100 ng of pcDV₁ fragment DNA, 1.0 $\mu$l of 10 $\times$ T4 ligase buffer in distilled water), and the reaction was performed overnight at 16°C. A half volume of the reaction mixture was used to transform E. coli HB101.

Twelve colonies out of the transformants obtained were used for plasmid DNA preparation by the mini-prep method, followed by digestion with various restriction enzymes. As a result, it was confirmed that all colonies contained the desired plasmid. Therefore, one strain among them was used for large-quantity preparation of the plasmid DNA, which was named pSV-G₁. The restriction enzyme cleavage map of pSV-G₁ was constructed (Fig. 3) and, as a result, it could be concluded that, in the plasmid pSV-G₁ obtained, the early enhancer/promoter element and the late 5'-splicing junction are located upstream from the KpnI site, which is the cloning site, and the late poly(A) signal and the terminator are positioned downstream therefrom. Hence, the elements involved in functional mRNA synthesis in animal cells are disposed in said plasmid without any deletion.

(3) Insertion of the pre-UK cDNA into a plasmid

The above-mentioned pUK33 (containing the urokinase gene about 2.2 kbp in total length) was partially digested with PstI, and a 1.7 kbp fragment was isolated and inserted into pSV-G₁. For the digestion; 100 $\mu$l of reaction mixture No. 4 (10 $\mu$g of pUK33, 10 $\mu$l of 10 $\times$ PstI buffer, 1 $\mu$l (6 U) of PstI in distilled water) was used and the reaction was carried out at 37°C. After 0, 5, 10, 15, 20, 40 and 60 minutes of digestion, the reaction mixture was sampled. The samples were subjected to 1.5% agarose gel electrophoresis to isolate the 1.7 kbp fragment. Then, 500 $\mu$l of reaction mixture No. 5 (10 $\mu$g of pUK33, 50 $\mu$l of 10 $\times$ PstI buffer, 10 $\mu$l (60 U) of PstI in distilled water) was maintained at 37°C and, after 10, 15 and 20 minutes of digestion, sampled in about 160 $\mu$l portions. After heat treatment at 65°C for 5 minutes, the reaction mixture samples were combined and subjected to 1% agarose gel electrophoresis to recover about 10 $\mu$g of the 1.7 kbp fragment. About 5 $\mu$g of the thus-recovered fragment of about 1.7 kbp was treated with T4 polymerase for conversion of the cleavage ends to blunt ends, ligated with a KpnI linker, and then ligated with KpnI-digested pSV-G₁. Transformation of E. coli HB101 with the ligation product and the subsequent conventional procedure gave pSV-G₁-pre-UK (Fig. 4).

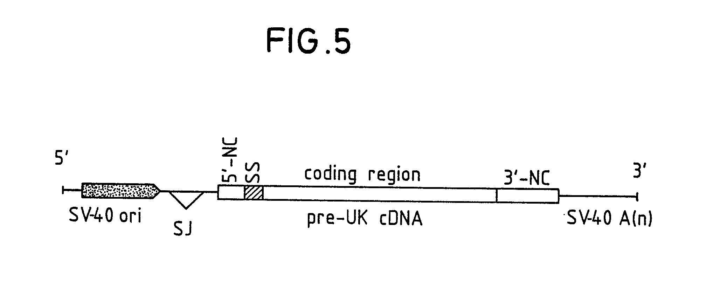

As shown in Fig. 5, pSV-G₁-pre-UK has the 5'-noncoding region, signal sequence and 3'-noncoding region of the pre-UK cDNA as inserted therein downstream from the mRNA transcription regulating region of SV40. Hence, this plasmid can produce human pro-UK when the DNA is introduced into appropriate recipient cells.

For reasons of convenience for treatment, this pSV-G₁-pre-UK was modified by converting the Acc I site to a HindIII site to give pSV-G₂-pre-UK.

(4) Insertion of the dhfr gene

Levinson el al. (EP 117060A), incorporated by reference herein, has succeeded in increasing the yield of TPA by incorporating the dhfr gene into plasmid vectors. the dhfr gene into plasmid vectors.

dhfr is the key enzyme for the de novo nucleic acid synthesis system and catalyzes the reduction of 7,8-dihydrofolate to 5,6,7,8-tetrahydrofolate. Methotrexate (herein after abbreviated as "MTX"), which is a derivative of folic acid, has very high affinity for dhfr and inhibits normal enzymatic reactions. Therefore, de novo nucleic acid synthesis is suppressed in the presence of MTX to an extent such that the growth of animal cells is significantly inhibited. On the other hand, it is known cytogenetically that when such cells have acquired resistance to MTX as a result of exposure to high concentrations of MTX, the dhfr gene has been amplified by several hundred times in the cells (Taisaibo Idengaku (Genetics of somatic cells), edited by Yamane et al., Rikogakusha (1981)).

The present inventors, too, aimed to increase the yield of pro-UK by making use of this dhfr gene.

The pSV2-dhfr plasmid (ATCC 37146) shown in Fig. 6 was used as the dhfr gene. An Xbal linker was inserted into this plasmid at the EcoRI site thereof to obtain pSV11-dhfr, and this was ligated with pSV-G₂-pre-UK carrying the pre-UK DNA to obtain pSVD-UK shown in Fig. 1.

## (5) Transformation

The method of Nagata et al., Tanpakushitu, Kakusan, Koso (Protein, Nucleic Acid, Enzyme), 28:(14), 1569-1581 (1983) was used.

## (6) Acquisition of·MTX resistant-host cells

(a) Resistance acquision by increasing the MTX concentration stepwise

$10^5$ cells of transformant with the plasmid DNA were inoculated into 25 cm² culture flasks (Falcon, 3013). MTX was added to a final concentration of 0.1 μM. When the cell growth in the presence of MTX became same rate as that attained in the absence of MTX, the cells were regarded as being resistant to 0.1 μM MTX. At the time of each transfer to a fresh medium, the MTX concentration was increased.

(b) Single step acquisition of MTX resistance

Instead of increasing the MTX concentration stepwise, the transformant was cultured in a high concentration of MTX from the beginning. Thus, $10^4$ cells of transformant were inoculated in culture dishes (100 mm in diameter, Falcon, 3003). MTX was added to a final concentration. of 0.05, 0.1, 0.5 or 2 μM. Medium exchange was conducted once a week.

## (7) Measurement of urokinase activity in culture supernatants

The cells (5 × $10^5$ cells/dish) were inoculated in dishes (100 mm in diameter). The supernatants collected after 3 days of cultivation were used as samples for urokinase activity measurement. The assay was performed by the fibrin plate method. Urokinase reference standard (Green Cross Corporation, 1150 IU/ampul) was used as a standard.

The following non-limiting examples are given for the purpose of illustrating the invention in further detail. A number of techniques, reaction procedures and analytical methods, which are applicable to the practice of the invention, are well known in the art. Unless otherwise specified, the enzymes used are available from commercial sources, such as Takara Shuzo; New England Biolabs (NEB), Massachusetts, USA; Amersham, England; and Bethesda Research Laboratories (BRL), Maryland, USA.

Unless otherwise specified, the buffers and reaction conditions for enzymatic reactions were as recommended by the manufacturers of the enzymes.

## EXAMPLE

### (1) Preparation of pSV11-dhfr

The plasmid pSV2-dhfr deposited under ATCC 37146 was used as the starting material. This was digested with Eco RI and an Xbal linker was inserted into the cleavage site. The thus-obtained pSV11-dhfr gene has a basic structure as shown in Fig. 1 with the dhfr gene inserted between the SV40 early promoter region and the SV40 polyadenylation site.

## (2) Preparation of pSV-G₂-pre-UK

pSV-G₂-pre-UK was prepared by using the plasmid pSV-G₁-pre-UK (5.0 kbp) disclosed in EP 154272A as the starting material and cleaving this at the AccI site and converting said site to a HindIII site.

## (3) Preparation of pSVD-UK

pSV-G₂-pre-UK was digested with HindIII, and a DNA fragment containing the urokinase gene was isolated by 1% agarose gel electrophoresis. An XbaI linker was joined to each end of the DNA fragment obtained. This was digested with XbaI. Separately, pSV11-dhfr was digested with XbaI and then subjected to DNA dephosphorylation with bovine small intestine-derived alklaline phosphatase. Both the digestion products were ligated together. The thus-obtained pSVD-UK was 7.7 kbp long, and the 1% agarose gel electrophoretic pattern obtained after BgIII digestion was in agreement with the restriction enzyme cleavage map shown in Fig. 1 with the urokinase gene and the dhfr gene inserted in opposite directions.

## (4) Transformation

DXB-11 cells were transformed with pSVD-UK by the DNA-calcium phosphate coprecipitation method.

Transformant selection was performed in Dulbecco's modified Eagle medium (DMEM) supplemented with proline (35 mg/1) and 10% fetal calf serum (FCS). On the 10th day after the start of selection and thereafter, colony appearance was observed. Colonies were picked yp by the cloning cylinder method and cultured in wells of 96-well plates (Falcon, 3072).

## (5) Production of urokinase

From among the transformants growing in wells of 96-well plates, 8 clones were selected for the first experiment of transformation and 13 clones for the second experiment, and the urokinase productions were measured. The results thus obtained are shown in Table 1. While most of the transformants obtained produced urokinase in an amount not more than 1 IU/ml, 6 transformant strainers showed urokinase productions of several IU/ml or more.

## Table 1

### UK production by cells with pSVD-UK introduced therein

| Cell line | | UK (IU/ml) |
|---|---|---|
| pSVD-UK1 | 131 | 3.5 |
| | 201 | 0 |
| | 202 | 0 |
| | 203 | < 0.39 |
| | 204 | < 0.39 |
| | 205 | < 0.39 |
| | 206 | 0 |
| | 213 | 3.3 |
| pSVD-UK2 | 101 | 0 |
| | 102 | 0 |
| | 103 | 0 |
| | 104 | 2.4 |
| | 105 | 0 |
| | 106 | 3.5 |
| | 201 | < 0.39 |
| | 203 | < 0.39 |
| | 204 | 4.6 |
| | 205 | < 0.39 |
| | 206 | 11 |
| | 207 | < 0.39 |
| | 208 | < 0.39 |

(6) Isolation of MTX resistant cells

(a) Increase in urokinase production as a result of stepwise acquisition of MTX resistance

An attempt was made to increase the yield of urokinase by increasing the MTX concentration in medium stepwise. In this experiment, pSVD-UKI-131 and pSVD-UKI-213 (cf. Table 1) were used. The starting MTX concentration used was 0.1 $\mu$M. Growth of pSVD-UK1-131 and pSVD-UK1-213 was not inhibited by 0.1 $\mu$M of MTX, hence were already resistant to 0.1 $\mu$M of MTX, at that concentration. Therefore, one week later, the MTX concentration was increased to 0.2 $\mu$M. Cell growth was also observed and therefore, another week later, the MTX concentration was increased to 0.5 $\mu$M. Thereafter, while confirming the cell growth by microscopy, the MTX concentration in the medium was increased to thereby increase the level of resistance. It took about 4 months to finally obtain resistance to 2 $\mu$M MTX. The thus-obtained resistant cells were examined for their ability to produce urokinase. The results obtained are shown in Table 2. As is evident from Table 2. It has been found that increases in resistance result in increased urokinase yields.

## Table 2

### Urokinase production by cells obtained by stepwise MTX resistance acquisition treatment

| Cell line | UK (IU/10$^6$ cells) | | |
|---|---|---|---|
| | – MTX | 1 $\mu$M MTX | 2 $\mu$M MTX |
| pSVD-UK1-131 | 9.5 | 32.8 | 30.0 |
| pSVD-UK1-213 | 2.9 | 11.3 | 15.9 |

(b) Increase in urokinase production as a result of single step MTX acquisition

Single step MTX acquisition was attempted using pSVD-UK2-206 cells (cf. Table 1). In this case, it was desired to increase the UK production by causing cells to acquire resistance in the presence of MTX at a high concentration from the beginning, not yet increasing the MTX concentration stepwise. 10$^5$ cells of pSVD-UK2-206 were inoculated in dishes (100 mm in diameter) and MTX were added to a final concentration of 0.05, 0.1, 0.5 and 2 $\mu$M. When cultivation was carried out in the presence of 0.05 and 0.1 $\mu$M MTX, MTX resistant colonies appeared in about 2 weeks. On the other hand, at 0.5 and 2 $\mu$M, MTX resistant colonies were found to appear in about 4-6 weeks. In this way, cells resistant to the respective concentrations of MTX were obtained, and they were evaluated for UK production. As shown in Table 3, resistant cell lines obtained at higher MTX concentrations showed higher productions.

## Table 3

### Urokinase production by cells obtained by single step MTX resistance acquisition treatment (IU/10$^6$ cells)

| – MTX | 0.05 $\mu$M MTX | 0.1 $\mu$M MTX | 0.5 $\mu$M MTX | 2 $\mu$M MTX |
|---|---|---|---|---|
| 9.9 | 14.3 | 34.3 | 49.6 | 50.9 |

Cell line: pSVD-UK2-206
Urokinase production: Given in terms of activity per 10$^6$ cells (measurement method being the same as above mentioned)

### (7) Identification of glycosylated single-chain pro-UK

The molecular weight of the human urokinase-like protein produced by the transformants, pSVD-UK2-206, was determined in the following manner.

The culture supernatant collected after 24 hours of culturing was subjected to 12.5% SDS-PAGE, protein bands were adsorbed electrophoretically onto a nitrocellulose filter in 25 mM Tris-192 mM glycine (pH 8.3)/20% methanol, and Western blotting was performed.

The filter was thereafter treated with 20 mM Trishydrochloride (pH 7.5)/500 mM sodium chloride containing 3% gelatin at room temperature for 60 minutes for blocking. Then, the human urokinase band was stained specifically with the Immuno-Blot™ assay system (Bio-Rad) using HRP (horseradish peroxidase) by the indirect method using, as a primary antibody, rabbit anti-human urokinase IgG purified on a human urokinase antigen column and protein A Sepharose.

With both the clones mentioned above, the culture supernatant, when treated for reduction with 2-mercaptoethanol, gave three bands, the main one corresponding to a molecular weight of 54 K and secondary two corresponding to molecular weights of 34 K and 20 K, respectively. Simultaneous electrophoresis of human urine-derived double-chain high molecular weight urokinase and a pro-UK producing fetal human kidney cell culture supernatant as controls gave a band (54 K) of human natural single-chain pro-UK and two bands (34 K and 20 K) of double-chain high molecular weight urokinase species, and these bands could be distinguished from but were in agreement with the three bands previously mentioned both in molecular weight and in reactivity with antiurokinase IgG. These results demonstrate that single-chain pro-UK, glycosylated in the same manner as said natural form, and having a molecular weight of 54 K, is synthesized and secreted by both the clones, with part of the pro-UK being secondarily converted to the double-chain forms in the culture medium.

Accordingly, the human urokinase-like protein synthesized and secreted by both the clones was examined for the presence or absence of sugar chains therein by column chromatography on ConA-Sepharose 4B (Pharmacia).

Cells were grown until confluent, washed twice with Dulbecco's PBS (+) (Nissui) and, then, cultured in 5 ml of MEM medium (1% FCS dialyzed against PBS (+), 35 mg/1 of proline, methionine-free, containing 200 μCi/ml of [$^{35}$S]-methionine) for 20 hours.

The $^{35}$S-labeled culture was applied to an anti-human urokinase monoclonal antibody column (5 ml). The column was washed thoroughly with 0.04 M sodium phosphate buffer (pH 8.0)/0.03 M sodium chloride and then eluted with glycine hydrochloride (pH 2.5), and $^{35}$S-labeled recombinant human urokinase was recovered. The $^{35}$S-labeled recombinant human urokinase-containing eluate was neutralized with 1 M Tris, then dialyzed against 20 mM sodium phosphate buffer (pH 7.4)/150 mM sodium chloride, and applied to a ConA-Sepharose 4B column equilibrated with the same buffer.

After washing the column with the buffer, elution was carried out with sodium phosphate buffer (pH 7.4) containing 0.1 M methyl-α-D-mannoside. Most of the radioactivity eluted from the anti-human urokinase monoclonal antibody column was adsorbed on ConA-Sepharose 4B and specifically eluted with the mannoside.

These results indicate that the recombinant urokinase synthesized and secreted by the clones obtained in accordance with the invention is a glycoprotein that can be purified on an anti-human urokinase monoclonal antibody.

The above results also indicate that the human pre-UK cDNA introduced into DXB-11 cells is transcribed in a normal manner and that the gene product is synthesized and secreted as a glycoprotein having the same molecular weight and the same reactivity with the monoclonal antibody as the natural form has.

While the invention has been described in detail and with reference to specific embodiments thereof, it will be apparent to one skilled in the art that various changes and modifications can be made therein without departing from the spirit and scope thereof.

### Claims

1. A method of producing glycosylated single-chain prourokinase which comprises cultivating dihydrofolate reductase gene-deficient CHO-K1 cells transformed with a plasmid containing, as inserts, a cDNA sequence shown below, SV40 promoter genes, and the dihydrofolate reductase gene, wherein the expression of said cDNA is controlled by a SV40 promoter gene and the expression of said dihydrolate reductase gene is controlled by a second SV40 promoter gene.

```
 1                              10                                  20
Ser-Asn-Glu-Leu-His-Gln-Val-Pro-Ser-Asn-Cys-Asp-Cys-Leu-Asn-Gly-Gly-Thr-Cys-Val-
AGC AAT GAA CTT CAT CAA GTT CCA TCG AAC TGT GAC TGT CTA AAT GGA GGA ACA TGT GTG

21                             30                                  40
Ser-Asn-Lys-Tyr-Phe-Ser-Asn-Ile-His-Trp-Cys-Asn-Cys-Pro-Lys-Lys-Phe-Gly-Gly-Gln-
TCC AAC AAG TAC TTC TCC AAC ATT CAC TGG TGC AAC TGC CCA AAG AAA TTC GGA GGG CAG

41                             50                                  60
His-Cys-Glu-Ile-Asp-Lys-Ser-Lys-Thr-Cys-Tyr-Glu-Gly-Asn-Gly-His-Phe-Tyr-Arg-Gly-
CAC TGT GAA ATA GAT AAG TCA AAA ACC TGC TAT GAG GGG AAT GGT CAC TTT TAC CGA GGA

61                             70                                  80
Lys-Ala-Ser-Thr-Asp-Thr-Met-Gly-Arg-Pro-Cys-Leu-Pro-Trp-Asn-Ser-Ala-Thr-Val-Leu-
AAG GCC AGC ACT GAC ACC ATG GGC CGG CCC TGC CTG CCC TGG AAC TCT GCC ACT GTC CTT

81                             90                                  100
Gln-Gln-Thr-Tyr-His-Ala-His-Arg-Ser-Asp-Ala-Leu-Gln-Leu-Gly-Leu-Gly-Lys-His-Asn-
CAG CAA ACG TAC CAT GCC CAC AGA TCT GAT GCT CTT CAG CTG GGC CTG GGG AAA CAT AAT

101                            110                                 120
Tyr-Cys-Arg-Asn-Pro-Asp-Asn-Arg-Arg-Arg-Pro-Trp-Cys-Tyr-Val-Gln-Val-Gly-Leu-Lys-
TAC TGC AGG AAC CCA GAC AAC CGG AGG CGA CCC TGG TGC TAT GTG CAG GTG GGC CTA AAG

121                            130                                 140
Pro-Leu-Val-Gln-Glu-Cys-Met-Val-His-Asp-Cys-Ala-Asp-Gly-Lys-Lys-Pro-Ser-Ser-Pro-
CCG CTT GTC CAA GAG TGC ATG GTG CAT GAC TGC GCA GAT GGA AAA AAG CCC TCC TCT CCT

141                            150                                 160
Pro-Glu-Glu-Leu-Lys-Phe-Gln-Cys-Gly-Gln-Lys-Thr-Leu-Arg-Pro-Arg-Phe-Lys-Ile-Ile-
CCA GAA GAA TTA AAA TTT CAG TGT GGC CAA AAG ACT CTG AGG CCC CGC TTT AAG ATT ATT

161                            170                                 180
Gly-Gly-Glu-Phe-Thr-Thr-Ile-Glu-Asn-Gln-Pro-Trp-Phe-Ala-Ala-Ile-Tyr-Arg-Arg-His-
GGG GGA GAA TTC ACC ACC ATC GAG AAC CAG CCC TGG TTT GCG GCC ATC TAC AGG AGG CAC

181                            190                                 200
Arg-Gly-Gly-Ser-Val-Thr-Tyr-Val-Cys-Gly-Gly-Ser-Leu-Ile-Ser-Pro-Cys-Trp-Val-Ile-
CGG GGG GGC TCT GTC ACC TAC GTG TGT GGA GGC AGC CTC ATC AGC CCT TGC TGG GTG ATC
```

```
201                              210                              220
Ser-Ala-Thr-His-Cys-Phe-Ile-Asp-Tyr-Pro-Lys-Lys-Glu-Asp-Tyr-Ile-Val-Tyr-Leu-Gly-
AGC GCC ACA CAC TGC TTC ATT GAT TAC CCA AAG AAG GAG GAC TAC ATC GTC TAC CTG GGT

221                              230                              240
Arg-Ser-Arg-Leu-Asn-Ser-Asn-Thr-Gln-Gly-Glu-Met-Lys-Phe-Glu-Val-Glu-Asn-Leu-Ile-
CGC TCA AGG CTT AAC TCC AAC ACG CAA GGG GAG ATG AAG TTT GAG GTG GAA AAC CTC ATC

241                              250                              260
Leu-His-Lys-Asp-Tyr-Ser-Ala-Asp-Thr-Leu-Ala-His-His-Asn-Asp-Ile-Ala-Leu-Leu-Lys-
CTA CAC AAG GAC TAC AGC GCT GAC ACG CTT GCT CAC CAC AAC GAC ATT GCC TTG CTG AAG

261                              270                              280
Ile-Arg-Ser-Lys-Glu-Gly-Arg-Cys-Ala-Gln-Pro-Ser-Arg-Thr-Ile-Gln-Thr-Ile-Cys-Leu-
ATC CGT TCC AAG GAG GGC AGG TGT GCG CAG CCA TCC CGG ACT ATA CAG ACC ATC TGC CTG

281                              290                              300
Pro-Ser-Met-Tyr-Asn-Asp-Pro-Gln-Phe-Gly-Thr-Ser-Cys-Glu-Ile-Thr-Gly-Phe-Gly-Lys-
CCC TCG ATG TAT AAC GAT CCC CAG TTT GGC ACA AGC TGT GAG ATC ACT GGC TTT GGA AAA

301                              310                              320
Glu-Asn-Ser-Thr-Asp-Tyr-Leu-Tyr-Pro-Glu-Gln-Leu-Lys-Met-Thr-Val-Val-Lys-Leu-Ile-
GAG AAT TCT ACC GAC TAT CTC TAT CCG GAG CAG CTG AAG ATG ACT GTT GTG AAG CTG ATT

321                              330                              340
Ser-His-Arg-Glu-Cys-Gln-Gln-Pro-His-Tyr-Tyr-Gly-Ser-Glu-Val-Thr-Thr-Lys-Met-Leu-
TCC CAC CGG GAG TGT CAG CAG CCC CAC TAC TAC GGC TCT GAA GTC ACC ACC AAA ATG CTG

341                              350                              360
Cys-Ala-Ala-Asp-Pro-Gln-Trp-Lys-Thr-Asp-Ser-Cys-Gln-Gly-Asp-Ser-Gly-Gly-Pro-Leu-
TGT GCT GCT GAC CCA CAG TGG AAA ACA GAT TCC TGC CAG GGA GAC TCA GGG GGA CCC CTC

361                              370                              380
Val-Cys-Ser-Leu-Gln-Gly-Arg-Met-Thr-Leu-Thr-Gly-Ile-Val-Ser-Trp-Gly-Arg-Gly-Cys-
GTC TGT TCC CTC CAA GGC CGC ATG ACT TTG ACT GGA ATT GTG AGC TGG GGC CGT GGA TGT

381                              390                              400
Ala-Leu-Lys-Asp-Lys-Pro-Gly-Val-Tyr-Thr-Arg-Val-Ser-His-Phe-Leu-Pro-Trp-Ile-Arg-
GCC CTG AAG GAC AAG CCA GGC GTC TAC ACG AGA GTC TCA CAC TTC TTA CCC TGG ATC CGC

401                              410
Ser-His-Thr-Lys-Glu-Glu-Asn-Gly-Leu-Ala-Leu
AGT CAC ACC AAG GAA GAG AAT GGC CTG GCC CTC
```

2. The method as claimed in claim 1, wherein said plasmid has the structure of pSVD-UK.

3. The method as claimed in claim 1, wherein said CHO-K1 cell-derived, dihydrolate reductase gene-deficient cell is DXB-11.

4. The method as claimed in claim 1, wherein said cDNA is prepared by using human kidney cell line-derived mRNA as template.

# FIG.1

FIG.2

# FIG.3

SalI
ClaI
HindIII
SphI
SphI
BglI
XhoI
HpaI
BamHI

PstI
BglI

Amp$^r$

SV40 ori

pSV-G1
(3.25 kbp)

SV-40 A(n)

KpnI
BamHI/XhoI
HpaI

pBR ori

AccI
BclI

# FIG.4

SalI  HindIII

E
P
Sj

KpnI
BglI
PstI

Amp$^r$   SV-40 ori

PstI

pSV-G1-preUK
(5.0 kbp)

pre-UK cDNA

SV-40 A(n)

AccI

BclI   KpnI   BamHI

# FIG.6

Amp$^r$

pBR322
ori

EcoRI

PstI

pSV2-dhfr

BamHI

Pvu II

SV40 ori   HindIII

BglII

Mo-dhfr

0 265 874

# FIG.5

5′            5′-NC   SS    coding region       3′-NC      3′

SV-40 ori    SJ       pre-UK cDNA         SV-40 A(n)